# EUROPEAN PATENT APPLICATION

(11) **EP 2 666 496 A1**
(43) Date of publication of application: **27.11.2013**
(21) Application number: 13168863.2
(22) Date of filing: 23.05.2013
(51) Int. Cl.: A61M 5/315, B65D 51/00

(54) **A rubber molded article**

(30) Priority: 25.05.2012 JP 2012120008
(71) Applicant: Daikyo Seiko, LTD., Tokyo 131-0031 (JP)
(72) Inventor: Sudo, Masamichi, Tokyo, Tokyo 131-0031 (JP)
(74) Representative: TBK

(57) **Abstract**

A rubber molded article which has a good visibility, prevents medical errors effectively by easily and certainly identifying injector or other containers containing medicinal agents which are used together with colored rubber molded articles, and prevents problems caused by dispersion of coloring agents into medicinal agents without harming the inherent properties of rubber molded articles like sealing performance.

A colored film layer 4 is positioned in the interior portion and a transparent resin portion 5 extends from at least one surface of the colored film layer 4 to the outermost layer.

## Description

### Technical Field

The present invention relates to a rubber molded article which is used for a container or an instrument for medicines, medical care, food products, cosmetic products, etc., especially a rubber molded article having a colored portion in the interior portion.

### Background Art

It is known to use an injector or a container for medicinal or medical purpose which includes a colored component for safe handling, especially in view of visibility and distinguishability.

For example, it is proposed to color a rubber portion (a slip stopper) of a piston, a body portion or a head portion of an injector in order to prevent fatal mistakes by error by identifying the intended purpose by color coding (Patent Document 1).

It is also proposed to associate each of plural colors with each of plural types of medicinal agents respectively and fill plural injectors by the plural types of medicinal agents so that a number of persons involved in a medical organization can identify the injector in which the desired medicinal agent is filled without causing confusions when they handle plural prefilled injectors (Patent Document 2).

Also, it is proposed to form an injector slip stopper by stacking a tetrafluoroethylene (TFE) resin film layer on a rubber elastic body in which the whole surface of the portion contacting with a medicinal agent and the portion sliding on the inner surface of an outer cylinder of an injector is colored in order to improve the visibility of the tip position of the slip stopper even when the injector outer cylinder is formed by a material having a low light transmissibility (Patent Document 3).

However, in the aforementioned prior art configurations, there are problems which are caused by elution of a coloring agent included in a colored component into medicinal agents (medicinal solutions) or change of properties of medicinal agents (medicinal solutions) by reacting with the coloring agent when the medicinal agents (medicinal solutions) contact with the colored component where a portion of the component or the whole component of an injector is colored.

These problems also naturally exist in containers for medicinal agents (medicinal solutions) or containers or instruments which are used for food products or cosmetic products.
Patent Document 1: Japanese Registered Utility Model No. 3070924
Patent Document 2: Japanese Laid-open Utility Model Application, Jikkai H6-9648
Patent Document 3: Japanese Laid-open Patent Application, Tokukou H7-47045

### Disclosure of Invention

### Problems to be resolved by the Invention

The task of the present invention is to resolve the aforementioned problems and provide a rubber molded article which perfectly prevent elution of coloring agents into medicinal agents or food products and safely preserve medicinal agents or food products filled in a container even if the medicinal agents (medicinal solutions), food products or cosmetic products contact with the colored component.

### Means for solving the Problems

In order to resolve the aforementioned problems, a rubber molded article according to the present invention comprises a colored film layer positioned in an interior portion and an outer transparent resin member which extends from at least one surface of the colored film layer to the outermost layer.

The colored film layer may comprise plural layers, and when the colored film layer comprises plural layers, the colored film layers may be all concolor or heterochromatic. The colored film layers may be an appropriate combination of concolor film layers and heterochromatic film layers.

As the colored film, any of a transparent colored film, a translucent colored film and an opaque colored film can be used, and if the colored film layer comprises plural layers, an appropriate combination of a transparent colored film, a translucent colored film and an opaque colored film can be used in addition to the aforementioned appropriate combination of concolor film layers and heterochromatic film layers.

When a combination of heterochromatic film layers using transparent films or translucent films is used, orange color is yielded by a combination of a yellow film and a red film, purple color is yielded by a combination of a red film and a blue film, and green color is yielded by a combination of a yellow film and a blue film by preparing transparent films or translucent films of three primary colors, i.e., a yellow film, a red film and a blue film, for example. It is also possible to yield additional color variation by changing the number of stacked layers of each colored film.

In this patent application, the term of "transparent resin" may include both transparent resin and translucent resin, and when the term of "transparent resin" is simply used it can include translucent resin hereinafter.

The "transparent resin member" may be a transparent synthetic resin film, a translucent synthetic resin film, a transparent rubber member, a translucent rubber member, a transparent thermoplastic elastomer member a translucent thermoplastic elastomer member or an appropriate combination thereof (for example, a combination of a transparent rubber member and a transparent resin film, a combination of transparent thermoplastic elastomer member and a transparent resin film).

When the rubber molded article is used as a rubber portion (a slip stopper) of a piston incorporated in an injector, a rubber plug for a vial container or a seal plug for a container or other instruments for food products or cosmetic products, the aforementioned transparent resin member is constituted by synthetic resin which does not impair the sealing performance, the sliding performance and other performances which are based on the intrinsic rubber elastic properties.

In a rubber molded article according to the present invention, in addition to the aforementioned properties, the aforementioned transparent resin member may be constituted by synthetic resin which can perfectly prevent elution into infill or reaction with the infill, and therefore perfectly prevent change of properties of the transparent resin itself even if the transparent resin member contacts with the infill, e.g., medicinal agents (medicinal solutions), food products or cosmetic products prefilled or filled as need arises. It is also possible to constitute the transparent resin member by plural layers and form only the outermost layer by resin which perfectly prevents such elution and reaction.

As the transparent resin preventing elution and reaction, it is preferable to use inactive resin like fluorine series resin which is usually used in the outermost layer of a normal rubber molded article, especially the portion contacting with medicinal agents (medicinal solutions) or food products.

In a rubber molded article according to the present invention, an inactive resin like a fluorine resin film which is usually used for this type of rubber molded article can be additionally stacked on the aforementioned transparent resin member, namely the surface contacting with medicinal agents (medicinal solutions), food products or cosmetic products.

In a rubber molded article according to the present invention, as the material of a constituent member other than the colored film and the transparent resin member, it is possible to use a material having rubber elastic properties (hereinafter, referred as rubber elastic material) like various types of synthetic rubber which are usually utilized in normal rubber molded articles or thermoplastic elastomer, etc. as is.

Also, the rubber elastic material can be transparent, translucent or opaque. It also can be colored as long as the color is clearly distinguished from that of the colored film or it has an effect of accentuating the color of the colored film.

In a rubber molded article according to the present invention, it is also possible to intervene an adhesive material like polyolefin (for example, ultrahigh molecular weight polyethylene) in order to firmly adhere the colored film with the transparent resin member or these members with the rubber elastic member to form a laminate body. It is also possible to omit the additional usage of adhesive material by forming the aforementioned colored film itself by polyolefin (for example, ultrahigh molecular weight polyethylene) which has good adhesiveness with the rubber elastic material.

### Effect of Invention

In a rubber molded article according to the present invention, it is possible to easily visually recognize a colored film layer positioned in an interior portion through a transparent resin portion because the portion extending from at least one surface of the colored film layer to the outermost layer is formed by a transparent resin.

In a rubber molded article according to the present invention, it is also possible for observers to recognize the rubber molded article according to the present invention as like as a rubber molded article simply made of colored rubber depending upon arrangement of the colored film layer and configuration of the transparent resin member (for example, a transparent resin film layer as the outermost layer is positioned immediately on a colored film layer, a transparent resin film as the outermost layer is positioned on a not thick transparent resin layer which is positioned immediately on a colored film layer.

Additionally, in a rubber molded article according to the present invention, as a colored film layer is positioned in the interior portion of a rubber molded article, it is possible to prevent problems which would be caused by elution of coloring agent from the colored film into medicinal agents (medicinal solutions) or change of properties of the medicinal agents (medicinal solutions) by reacting with the medicinal agents (medicinal solutions), because the colored film does not contact with the medicinal agents (medicinal solutions) or food products filled in injectors, vial containers, other containers or instruments using a rubber molded article according to the present invention. Furthermore, it is possible to maintain a good colored condition for long time without concerns about antiweatherability or durability, because the colored film is positioned in the interior portion of the rubber molded article.

As a result of the aforementioned configurations, it is possible to easily and successfully associate the colors given to a rubber molded article according to the present invention by the colored films with the types of the medicinal agents (medicinal solutions) or food products prefilled or filled as need arises in injectors, vial containers, other containers or instruments which use a rubber molded article according to the present invention.

By this association, it is possible to easily and certainly identify by color the injector or the container in which the desired medicinal agent (medicinal solution) or food product is contained based on the good visibility and distinguishability. Therefore, it is possible to prevent fatal medical errors or dietary errors where incorrect medicinal agents (medicinal solutions) or food products are applied to human bodies.

### Brief Description of Drawings

Fig. 1 shows a configuration when a rubber molded article according to the present invention is a rubber portion (a slip stopper) of an injector piston.
Fig. 2 (A), (B) show other two configurations when a rubber molded article according to the present invention is a rubber portion (a slip stopper) of an injector piston.
Fig. 3 shows a configuration when a rubber molded article according to the present invention is a rubber plug for a vial container.
Fig. 4(A), (B) show other two configurations when a rubber molded article according to the present invention is a rubber plug for a vial container.

### A mode for implementing the Invention

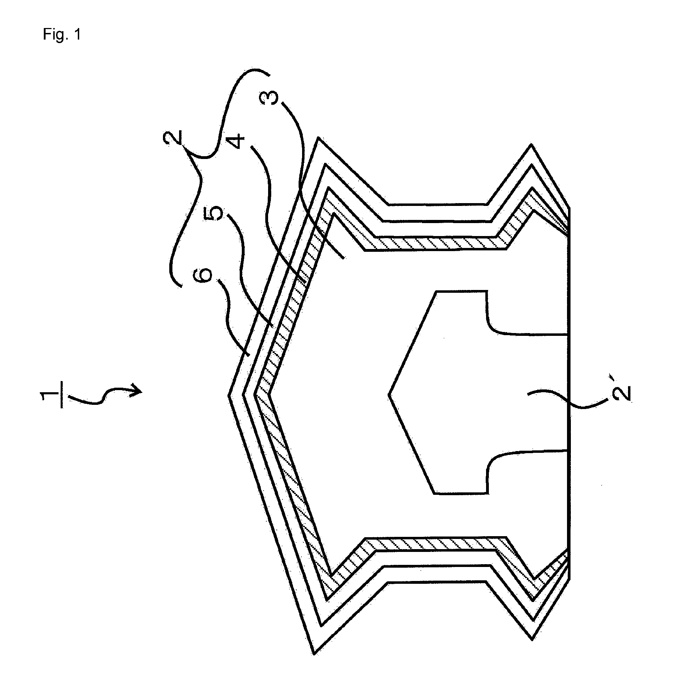

In Fig. 1, a rubber portion (a slip stopper) 1 for an injector piston comprises a slip stopper body 2 and a piston rod support portion 2' which is formed in the slip stopper body 2. In this configuration, the slip stopper body 2 comprises a slip stopper base member 3 which is formed by rubber elastic material, a colored film layer 4 which is formed on the slip stopper base member 3 and a transparent resin member 5 which is formed on the colored film layer 4.

In this configuration, a transparent film 6 made of inactive resin like fluorine series resin is stacked on the transparent resin member 5, and the body including the transparent film 6 is referred as the slip stopper body 2.

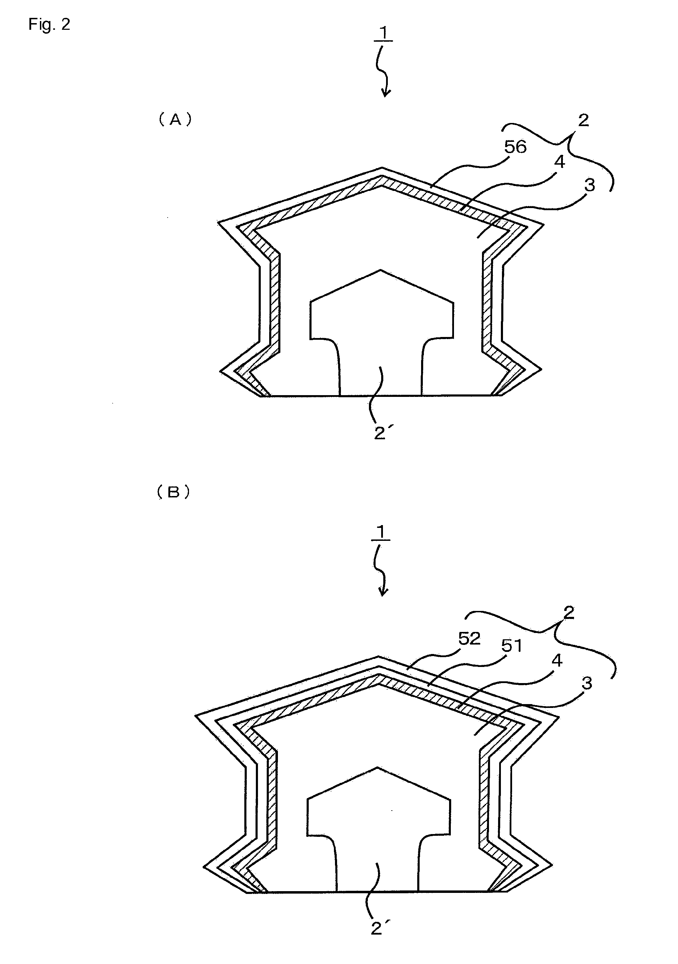

It is possible to replace the transparent resin member 5 (which is formed on the colored film layer 4) with a transparent resin member (film or sheet) 56 made of inactive resin like fluorine series resin as shown in Fig. 2 (A) without using the transparent film 6. It is also possible to constitute the transparent resin member 5 by plural layers, for example, two layers 51, 52 as shown in Fig. 2(B) and form only the outermost layer 52 by inactive resin like fluorine series resin.

Although it is not shown in drawings, it is possible to intervene an ultrahigh molecular weight polyolefin film as adhesive material between the colored film layer 4 and the transparent resin members 5, 56, 51, between the colored film layer 4 and the slip stopper base member 3, and between the transparent resin member 51 and the transparent resin member 52. It is also possible to form the colored film layer 4 itself by ultrahigh molecular weight polyolefin.

In the rubber portion (slip stopper) 1 of the piston shown in Fig. 1 and Fig. 2(A), (B) having the aforementioned configurations, the colored film layer 4 can be visually recognized through the transparent resin members 5, 56, 51, 52 or the stacked transparent film 6 made of the inactive resin. And, it is possible to recognize the color of the colored film layer 4 when the rubber portion (slip stopper) 1 of the piston is observed.

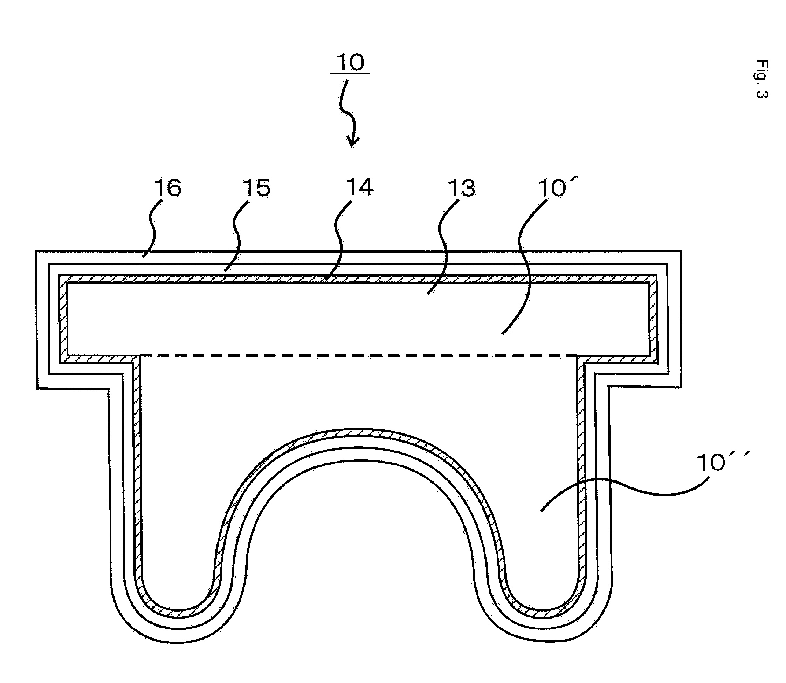

In Fig. 3, a rubber plug 10 for a vial container comprises a cap portion 10' and a leg portion 10", and in this configuration, the rubber plug 10 is formed by a plug body base member 13 made of rubber elastic material, a colored film layer 14 positioned on the plug body base member 13 and a transparent resin member 15 positioned on the colored film layer 14.

In this configuration, a transparent film 16 made of inactive resin like fluorine series resin is stacked on the transparent resin member 15.

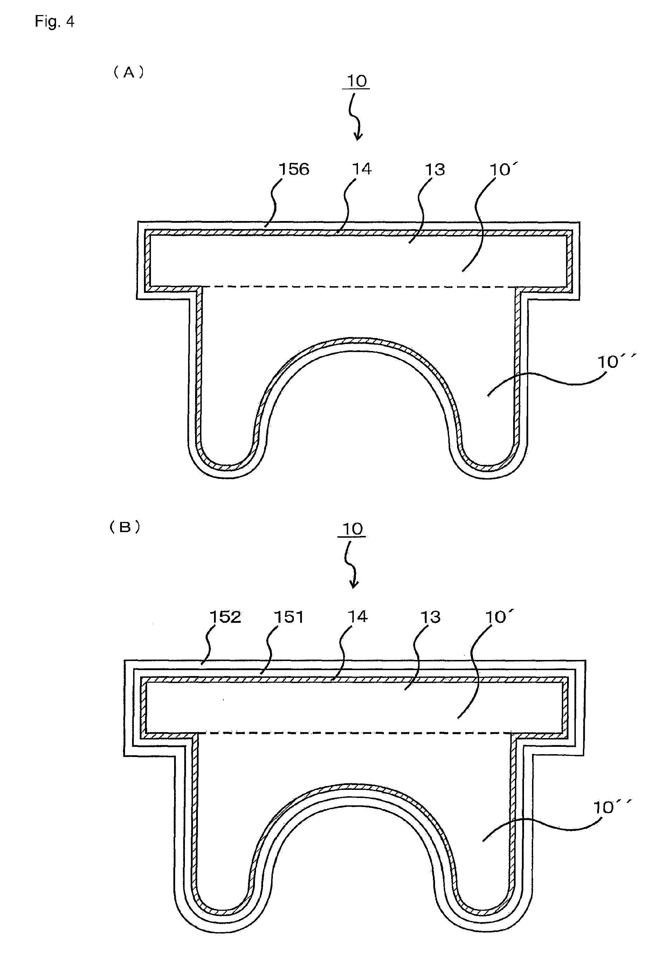

Also, in this configuration, it is possible to replace the transparent resin member 15 (which is positioned on the colored film layer 14) with a transparent resin member (film or sheet) 156 made of inactive resin like fluorine series resin as shown in Fig. 4 (A) without having the transparent film 16. It is also possible to constitute the transparent resin member 15 by plural layers, for example, two layers 151, 152 as shown in Fig. 4(B) and form only the outermost layer 152 by inactive resin like fluorine series resin.

Also, in these configurations, although it is not shown in drawings, it is possible to intervene an ultrahigh molecular weight polyolefin film as adhesive material between the colored film layer 14 and the transparent resin members 15, 156, 151, between the colored film layer 14 and plug body base member 13, and between the transparent resin member 151 and the transparent resin member 152. It is also possible to form the colored film layer 14 itself by ultrahigh molecular weight polyolefin.

In the configurations shown in Figs. 1 through 4, it is possible to constitute the colored film layer 4, 14 as plural layers, and constitute them by one or more selected from transparent films, translucent films and opaque films. It is also possible to use an opaque member colored in white, gray or other color as the slip stopper base member 3 and the plug body base member 13.

In the present invention, as described above, the slip stopper 1 and the rubber plug 10 comprise the rubber layers (rubber elastic members) 3, 13 and the outermost layers 6,56,52, 16, 156, 152 which are stacked on the portion of the rubber elastic member contacting with medicinal agents (medicinal solutions) and the whole surface of the portion sliding on the inner surface of the injector outer cylinder and the inner surface of the vial container opening portion, and the rubber elastic members 3, 13 (the slip stopper base member, the plug body base member) are colored so that the tip position of the slip stopper 1 and the leg portion 10" of the rubber plug 10 can be successfully recognized. The tip position of the slip stopper and the rubber plug leg portion can be easily visually recognized even if the injector outer cylinder and the vial container are made of a material which does not have a high transparency like glass, for example, polypropylene (PP), polyethylene (PE) or other plastic material which has a low light transmittance and a low transparency.

### Rubber Elastic Member:

In the present invention, as the rubber elastic members 3, 13, the main raw material can be synthetic rubber or natural rubber like isoprene rubber, butadiene rubber, styrene-butadiene rubber, ethylene-propylene rubber, isoprene-isobutylene rubber type, nitrile rubber, etc., and a filler and a cross-linking agent, etc., are compounded with it.

Also, thermoplastic elastomer can be used. Specifically, it is preferable to use at least one type selected from olefin series(TPO), styrene series (SBC), vinyl chloride series (TPVC), urethane series (TPU), polyester series (TPEE), polyamide (TPAE), fluorine series (TPF), polybutadiene series (RB), polyisobutylene series, silicone series, ethylene-vinyl acetate series (EVA,EEA), etc., having an intermediate characteristic between rubber and plastic. Among them, it is preferable to use styrene-ethylene-butadiene copolymer (SEBS), styrene-butadiene copolymer (SBS), styrene-isoprene copolymer (SIS), styrene-isobutylene copolymer (SIBS) etc. in view of a good heat resistance and a good elution characteristic.

The material of the rubber elastic members 3, 13 is selected from the aforementioned materials depending upon the molding condition and other conditions so that the color of the rubber material itself does not become blurred and the color unevenness is caused in the molded article.

### Outermost Layer:

It is preferable to use fluorine series resin having a low frictional resistance as the material of the outermost layers 6, 56, 52, 16, 156, 152 because it has a good sliding property, it has no risk of interfusing in a medicinal agents (medicinal solutions), it does not break away, and it can closely contact with the slip stopper. As the fluorine series resin, it is preferable to use polytetrafluoroethylene (PTFE), tetrafluoroethylene-perfluoroalkylvinyl ether copolymer (PFA), tetrafluoroethylene-ethylene copolymer (ETFE), tetrafluoroethylene-hexafluoropropylene copolymer (FEP), polychlorotrifluoroethylene (CTFE), polyvinylidene fluoride (VdF), trifluoroethylene-ethylene copolymer, polytetrafluorodioxole copolymer, polyvinyl fluoride, etc., which has a good light resistance and a good water vapor permeation resistance, etc. Among them, it is more preferable to use PTFE, PFA, ETTE, FEP, VdF in view of a good antifouling property and a good heat resistance, etc. Further preferably, PTFE can be used because it has a small friction coefficient, a high flexibility and a good sealing performance.

It is preferable to use PTFE in a form of a film having a thickness between 10 µm and 100 µm in view of a high flexibility and a good sealing property and stack it on a rubber elastic slip stopper base member.

As shown in drawings, the slip stopper 1 of an injection piston and the rubber plug 10 for a vial container in the aforementioned configurations comprise a slip stopper base member 3 and a plug body base member 13 respectively formed by rubber elastic material, and the outermost layers 6, 56, 52, 16, 156, 152 are stacked on the surface of the slip stopper base member 3 and the plug body base member 13 via the colored film layers 4, 14 respectively. A sufficient sliding property and a sufficient chemical resistance are obtained by forming the aforementioned outermost layers on the portion contacting with medicinal agents (medicinal solutions) and the whole surface of the portion sliding on the inner surface of the injector body outer cylinder and the inner surface of the vial container opening portion.

Also only a portion of the outermost surface layer can be made of inactive resin like fluorine series resin in place of the whole surface as need arises.

### [Embodiment 1]

The rubber plug 10 for a vial container shown in Fig. 4(A) was made by using the following materials and method.

As the rubber elastic member 13, a white color sheet body having a thickness of 10mm was prepared by mixing 100 pts.wt. of polyisobutylene thermoplastic elastomer (SIBSTAR ^{(R)} made by Kaneka Corp.) with 2 pts.wt. of a cross-linking agent (hydrosilyl group contained polysiloxane) using an open roll, aging for 24 hours and then heating. As the colored film 14, an ultrahigh molecular weight polyethylene film (Products Name "No. 440": molecular weight 5.5 million made by Nitto Denko Corp.) having a thickness of 50 µm and being mixed with a red colorant was prepared.

As the transparent resin member 156, an ETFE (ethylenetetrafluoroethylene resin) colorless transparent sheet body having a thickness of 100 µm (Product Name "NEOFLON™" made by Daikin Kogyo Corp.) was prepared.

The aforementioned ETFE colorless transparent sheet body as the transparent resin member 156 was put on the lower mold of a mold for forming a vial container rubber plug, and the aforementioned ultrahigh molecular weight polyethylene film as the colored film 14 and the aforementioned SIBS series white color sheet body as the rubber elastic member 13 were stacked on it in this order (these members will form the leg portion 10' of the rubber plug 10), and then a SIBS series white color sheet body as the rubber elastic member 13, an ultrahigh molecular weight polyethylene film as the colored film 14 and an ETFE colorless transparent sheet body as the transparent resin member 156 were stacked on the rubber elastic member (white color sheet body) 13 (these members will become the cap portion 10") in this order (which is the reverse order of the aforementioned order), and an upper mold tool was taken down.

The upper and lower mold tools were heated to 150-180°C, the mold clamping pressure was set to 100kg/cm², a heat pressure forming was performed for 10 minutes, and then the vial container rubber plug shown in Fig. 4(A) was obtained by cutting the formed article along cavities.

In this rubber plug, the red color of the colored film 14 was easily, certainly and finely visually recognized through the transparent resin member 156.

Also, a vial container was capped by this rubber plug, and heat-sterilized at 121°C for 30 minutes. The change in color was visually observed, and the adhesion condition (whether detachment occurred) between the cap portion 10' and the leg portion 10" and the adhesion condition between each of the layers (the transparent resin member 156, the colored film 14, the rubber elastic member 13) were visually observed, immediately after the capping, immediately after the heat-sterilization, immediately after cooling to room temperature and after keeping it for 24 hours at room temperature. The adhesion conditions between the portions and between the layers were also observed after continuously attempting to manually break away them using tweezers while the vial container is capped by the rubber plug.

In any result, no change in color was recognized, and no detachment was recognized by visual observation, and no detachment occurred between the cap portion 10' and the leg portion 10" and between each of the layers even by the manual peeling-off attempt.

### [Embodiment 2]

The rubber plug 10 for a vial container shown in Fig. 4(B) was made using the following materials and method.

As the transparent resin member 151, a white color translucent resin sheet body having a thickness of 1 mm was prepared by mixing 100 pts.wt. of SIBS series elastomer (SIBSTR^{(R)} made by Kaneka Corp.) with 2 pts.wt. of a cross-linking agent (hydrosilyl group contained polysiloxane) using an open roll, aging for 24 hours and then heating.

As the transparent resin member 152, an ETFE (ethylenetetrafluoroethylene resin) colorless transparent sheet (film) body having a thickness of 0.5mm (Product Name "NEOFLON™" made by Daikin Kogyo Corp.) was prepared.

As the rubber elastic member 13 and the colored film 14, the same things that were used in Embodiment 1 were prepared.

An ETFE colorless transparent sheet body as the transparent resin member 152 was put on the lower mold tool of a mold tool for forming a vial container rubber plug which is same as that used in Embodiment 1. A SIBS series white color translucent sheet body as the transparent resin member 151, an ultrahigh molecular weight polyethylene film as the colored film 14 and a SIBS series white color sheet body as the rubber elastic member 13 were stacked on the transparent resin member 152 in this order (these members will form the leg portion 10" of the rubber plug 10. And then, a SIBS series white color sheet body as the rubber elastic member 13, an ultrahigh molecular polyethylene film as the colored film 14, a SIBS series white color translucent sheet body as the transparent resin member 151 and an ETFE colorless transparent film as the transparent resin member 152 were stacked on the white color sheet body (rubber elastic member) 13 (these members will become the cap portion 10') in this order (which is the inverse order of the aforementioned order), and an upper mold tool was taken down.

The upper and lower mold tools were heated to 150-180°C, the mold clamping pressure was set to 100kg/cm², a heat pressure forming was performed for 10 minutes and then the vial container rubber plug shown in Fig.4(B) was obtained by cutting the formed article along cavities.

Also in this rubber plug, similarly to the rubber plug according to Embodiment 1, the color of the colored film 14 was easily, certainly and finely visually recognized through the translucent synthetic resin member 151 and the transparent synthetic resin member 152. However the colored film 14 was observed as having a little bit whity-red color with a unique tint in place of the brilliant red color of the material because it is observed through the white color translucent synthetic resin layer 151.

The change in color and the adhesion condition were observed for this rubber plug similarly to the rubber plug according to Embodiment 1 and a very good result which was similar to that of the rubber plug according to Embodiment 1 was obtained.

### [Embodiment 3]

The rubber portion (slip stopper) 1 of a piston shown in Fig. 2(A) was made using the following materials and method.

As the rubber elastic member 3, the colored film 4, the transparent resin member 56, the same things as those used in Embodyment 1, as the rubber elastic member 13, the colored film 14 and the transparent resin member 156 were prepared.

An ETFE colorless transparent sheet body as the transparent resin member 56 was put on the lower mold tool (a recess for forming the apical projection of the slip stopper 1 in Fig. 2(A) is formed) of a mold tool for a piston rubber portion (a slip stopper). An ultrahigh molecular weight polyethylene film as the colored film 4 and a SIBS series white color sheet body as the rubber elastic member 3 were stacked on the transparent resin member 56 in this order. A slip stopper shown in Fig. 2(A) was obtained by applying a compression molding by pressurizing and heating at a mold clamping pressure of 100kg/cm² and 180°C for 7 minutes using an upper mold tool (a convex portion for forming a piston rod supporting portion 2' is formed), and by punching out burr portions.

In this slip stopper, the red color of the colored film 4 was easily, certainly and finely visually recognized through the transparent resin member 56.

Also, a piston rod (not shown in drawings) was fixed to the piston rod support portion 2' of this slip stopper, inserted into the outer cylinder of a vinyl chloride series resin made injector having a low transparency, and heat-sterilized at 121°C for 30 minutes. The change in color and the adhesion condition between each of the layers (the transparent resin member 56, the colored film 4 and the rubber elastic member 3) were visually observed immediately after insertion, immediately after heat-sterilization, immediately after cooling to room temperature and after keeping it for 24 hours at room temperature, respectively. The adhesion condition between each of the layers were also observed after continuously attempting to manually break away them using tweezers for one minute while the piston rod taken out from the outer cylinder was fixed.

As a result, the red color of the slip stopper was easily and certainly visually recognized through the outer cylinder even when it was in an injector outer cylinder having a low transparency. In any case, no change in color was recognized and no detachment was recognized by visual observation. No detachment occurred between each of the layers by the manual breaking away attempt using tweezers.

### [Embodiment 4]

The rubber portion (slip stopper) 1 of a piston shown in Fig. 2(B) was made using the following materials and method.

As the rubber elastic member 3, the colored film 4, the transparent resin members 51, 52, the same things as those used in Embodyment 2, as the rubber elastic member 13, the colored film 14, the translucent synthetic resin member 151 and the transparent resin member 152 were prepared.

An ETFE colorless transparent sheet body as the transparent resin member 52 was put on the lower mold tool of a mold tool for a piston rubber portion (a slip stopper) which is same as that used in Embodiment 3. A SIBS series white color translucent sheet body as the translucent resin member 51, an ultrahigh molecular weight polyethylene film as the colored film 4 and a SIBS series white color sheet body as the rubber elastic member 3 were stacked on the transparent resin member 52 in this order. A slip stopper shown in Fig. 2(B) was obtained by applying a compression molding by pressurizing and heating at a mold clamping pressure of 100kg/cm² and at 180°C for 7 minutes using the same upper mold tool as that used in Embodiment 3, and by punching out burr portions.

In this slip stopper, the color of the colored film 4 was easily, certainly and finely visually recognized through the translucent synthetic resin member 51 and the transparent synthetic resin member 52.

The colored film 14 was observed as having a little bit whity-red color with a unique tint in place of the brilliant red color of the material because it is observed through the white color translucent synthetic resin layer 51.

The change in color and the adhesion condition were also observed for this slip stopper similarly to the slip stopper according to Embodiment 2 and a very good result which was similar to that of the slip stopper according to Embodiment 3 was obtained.

### Field of Industrial Application

A rubber molded article according to the present invention can be preferably used as a rubber molded article which is used together with various types of medical instruments, e.g., a cap for a syringe needle attaching portion at the injector tip, an intravenous drip container, a dialysis liquid medicinal agent container, a needle sticking portion of transfusion plug body, a transfusion device, a blood collection instrument, a prosthetic device, a catheter, a blood pack, or bottles and other instruments for food products or cosmetic products in addition to the aforementioned rubber portion (a slip stopper) of an injector piston and the vial container rubber plug because the color applied by the colored film can be successfully and easily visually recognized.

In this case, it is possible to identify the type of infill easily and certainly based on a good visibility and a good distinguishability of a rubber molded article according to the present invention and prevent fatal medical errors or dietary errors where incorrect medicinal agents (medicinal solutions) or food products are applied to human bodies by associating a color of a rubber molded article according to the present invention with a type of medicinal agents (medicinal solutions), food products or cosmetic products which are prefilled or filled as need arises in the aforementioned injectors, vial containers or other various types of instruments..

A rubber molded article which has a good visibility, prevent medical errors effectively by easily and certainly identifying injector or other containers containing medicinal agents which are used together with colored rubber molded articles, and prevent problems caused by dispersion of coloring agents into medicinal agents without harming the inherent properties of a rubber molded article like sealing performance.

A colored film layer 4 is positioned in the interior portion and a transparent resin portion 5 extends from at least one surface of the colored film layer 4 to the outermost layer.

## Claims

1. A rubber molded article comprising a colored film layer positioned in the interior portion, and a transparent resin portion which extends from at least one surface of the colored film layer to the outermost layer.

2. A rubber molded article according to Claim 1, the colored film layer comprises plural layers, and the plural layers are concolor, heterochromatic or of a combination of concolor and heterochromatic.

3. A rubber molded article according to Claim 1 or 2, the colored film layer comprises one or more selected from transparent, translucent or opaque films.

4. A rubber molded article according to either one of Claims 1 through 3, the transparent resin portion comprises one or more selected from transparent resin, transparent rubber or transparent thermoplastic elastomer.
